# EUROPEAN PATENT APPLICATION

(11) **EP 3 332 717 A1**
(43) Date of publication of application: **13.06.2018**
(21) Application number: 16834838.1
(22) Date of filing: 05.04.2016
(51) Int. Cl.: A61B 17/068, A61B 17/125

(54) **TREATMENT DEVICE**

(30) Priority: 07.08.2015 JP 2015157043
(71) Applicant: Olympus Corporation, Hachioji-shi, Tokyo 192-8507 (JP)
(72) Inventor: HATANAKA, Takayuki, Hachioji-shi Tokyo 192-8507 (JP); SAKAMOTO, Tetsuyuki, Hachioji-shi Tokyo 192-8507 (JP)
(74) Representative: Schicker, Silvia
(86) International application number: PCT/JP2016/061175
(87) International publication number: WO 2017/026141

(57) **Abstract**

A treatment device (1) includes: an operating section (3); an elongated insertion section (2) connected to the operating section (3); a pair of clamp members (5 and 6) provided at the distal end of the insertion section (2) so as to be openable and closable relative to each other; a cutter provided in one clamp member (5); a movable member that is moved so as to push the cutter relative to tissue; and a driving mechanism for moving the movable member towards the distal end side. The driving mechanism includes: a coil member arranged so as to pass through the insertion section (2) in the longitudinal direction and transmits a pressing force from the operating section (3) to the movable member; and a guide wire both ends of which are fixed to the one clamp member (5) and to the operating section (3).

## Description

### {Technical Field}

The present invention relates to a treatment device.

### {Background Art}

There is a known treatment device that is operated under laparoscopy to grip, cut off, and staple tissue when a lesion in a digestive tract is resected in full thickness via lapa-combine (combination procedure between Endoscope and Laparoscope) (refer to, for example, Patent Literature 1).

The tissue is cut off and stapled using the treatment device in such a manner that a cutter and a wedge member are pushed along a pair of jaws towards the distal end side relative to the tissue sandwiched between the jaws.

When the wedge member is moved towards the distal end, a bevel thereof pushes pushers in a direction intersecting the moving direction of wedge member, and then the pushers push staples towards the tissue, thereby sequentially stapling the tissue towards the distal end. In addition, the cutter disposed behind the wedge member cuts, towards the distal end, the tissue that has just been stapled.

In Patent Literature 1, in order to produce a force for pushing the wedge member and the cutter along the curved jaws towards the distal end side, the wedge member and the cutter are fixed to one end of a wire that is folded back via a pulley disposed at the distal end of a jaw, and the other end of the wire is pulled at the operating section.

### {Citation List}

### {Patent Literature}

{PTL 1} Japanese Unexamined Patent Application, Publication No. 8-289895

### {Summary of Invention}

### {Technical Problem}

However, with a structure in which the wire is folded back via the pulley, it is necessary to secure a relatively large diameter of the pulley to prevent the wire from being curved with a small radius of curvature. Therefore, this is inconvenient in that the width of the distal end portion of the treatment device is large, resulting in a decrease in the ease of insertion of the treatment device into the living body.

The present invention has been conceived in light of the above-described circumstances, and an object thereof is to provide a treatment device that allows treatment, such as cutting off and stapling of tissue, to be carried out easily and reliably while still avoiding an increase in the size of the distal end portion.

### {Solution to Problem}

In order to achieve the above-described object, the present invention provides the following solutions.

A first aspect of the present invention is a treatment device including: an operating section operated by an operator; an elongated insertion section that is connected to the operating section and that has flexibility; a stitching section that is provided at a distal end of the insertion section and that includes a first clamp member that accommodates a plurality of arranged staples and a second clamp member that is provided so as to be openable and closable relative to the first clamp member; a movable member that is provided in the first clamp member of the stitching section and that is moved so as to push the staples relative to a tissue sandwiched between the first clamp member and the second clamp member; and a driving mechanism for moving the movable member towards a distal end side, wherein the driving mechanism includes: a coil member that is provided so as to pass through the insertion section in a longitudinal direction, the distal end of which is brought into contact with the movable member, and that transmits a pressing force applied in the operating section; and a guide wire that passes through the coil member in the longitudinal direction, one end of which is fixed to the first clamp member and the other end of which is fixed to the operating section, and that guides the movement of the coil member.

According to the first aspect, the insertion section is inserted into a hole provided in the abdominal wall, the body cavity, or a lumen, the stitching section at the distal end is placed inside the body, the second clamp member is closed relative to the first clamp member, and the driving mechanism is operated by operating the operating section in a state where the tissue is sandwiched between the clamp members. The pressing force applied in the operating section is transmitted to the movable member by the coil member constituting the driving mechanism, and the movable member is moved as a result of being pushed towards the distal end side along the longitudinal direction of the insertion section. By doing so, the tissue is stapled as a result of the distal end member pushing, towards the tissue, the staples accommodated in the first clamp member.

In this case, because the coil member is guided by the guide wire passing therethrough, the coil member is held by the guide wire so as not to buckle even when a large pressing force is applied to the coil member. In addition, when the insertion section is bent, the wire and the coil member can be bent just as the insertion section is bent, thus making it possible to transmit the pressing force by means of the coil member even in a state where the insertion section is bent. In addition, even if the first clamp member and the second clamp member are shaped so as to bend along the longitudinal direction, the movable member can be moved by reliably transmitting the pressing force by means of the coil member.

Then, as a result of using such a coil member, it is not necessary to provide, at the distal end of the stitching section, a pulley for folding back the wire, thereby preventing the stitching section from having a large diameter. This enhances the ease of insertion into the body and consequently reduces stress to the patient.

A second aspect of the present invention is a treatment device including: an operating section operated by an operator; an elongated insertion section that is connected to the operating section and that has flexibility; a stitching section including a first clamp member that is provided at a distal end of the insertion section and that accommodates a plurality of arranged staples and a second clamp member that is provided so as to be openable and closable relative to the first clamp member; a cutter for cutting a tissue sandwiched between the first clamp member and the second clamp member of the stitching section; a movable member that is provided in the first clamp member of the stitching section and that is moved so as to push either or both of the staples and the cutter relative to the tissue sandwiched between the first clamp member and the second clamp member; a driving mechanism for moving the movable member towards a distal end side, wherein the driving mechanism includes: a coil member that is provided so as to pass through the insertion section in a longitudinal direction, the distal end of which is brought into contact with the movable member, and that transmits a pressing force applied in the operating section; and a guide wire that passes through the coil member in the longitudinal direction, one end of which is fixed to the first clamp member and the other end of which is fixed to the operating section, and that guides the movement of the coil member.

According to the second aspect, the insertion section is inserted into a hole provided in the abdominal wall, the body cavity, or a lumen, the stitching section at the distal end is placed inside the body, the second clamp member is closed relative to the first clamp member, and the driving mechanism is operated by operating the operating section in a state where the tissue is sandwiched between the clamp members. The pressing force applied in the operating section is transmitted to the movable member by the coil member constituting the driving mechanism, and the movable member is moved as a result of being pushed towards the distal end side along the longitudinal direction of the insertion section. By doing so, the tissue is stapled and cut as a result of the distal end member pushing, towards the tissue, either or both of the staples and the cutter accommodated in the first clamp member.

The above-described first and second aspects may include a hollow tube that has flexibility and that allows the coil member to pass therethrough so as to be movable in the longitudinal direction in the insertion section.

By doing so, even though the coil member is widening radially outward due to a compression force applied to the coil member when the pressing force is transmitted by means of the coil member, the widening is suppressed by the hollow tube through which the coil member passes, thereby more reliably preventing the coil member from buckling.

In the above-described first and second aspects, the first clamp member may accommodate the plurality of staples such that the staples are arranged in the longitudinal direction of the insertion section, and the movable member may be moved towards the distal end side along the first clamp member so as to push out the staples sequentially.

By doing so, in the treatment device of a so-called linear method in which the staples are accommodated in the first clamp member so as to be arranged along the longitudinal direction of the insertion section, stapling can be performed by sequentially pushing out the staples towards the tissues.

In the above-described first and second aspects, the first clamp member may include a guide groove for guiding the movement of the movable member.

By doing so, because the movement of the movable member is guided by the guide groove, the movable member can be moved smoothly towards the distal end of the first clamp member merely by applying the pressing force transmitted by means of the coil member.

In the above-described first and second aspects, the movable member may include a slider section that slides in the guide groove, and the slider section may be shaped so as to be in contact with each side wall of the guide groove at two positions with a space interposed therebetween in the slide direction of the slider section.

By doing so, even if the guide groove is curved, the movable member can be guided reliably by causing the slider section to be in touch with each side wall of the guide groove at two positions.

In the above-described first and second aspects, the first clamp member may accommodate the plurality of staples such that the staples are arranged on an arc along a direction orthogonal to the longitudinal direction of the insertion section, and the movable member may be moved so as to push out the staples all at once.

By doing so, in the treatment device of a so-called circular method in which the staples are accommodated in the first clamp member so as to be arranged in an arc shape along a direction orthogonal to the longitudinal direction of the insertion section, stapling can be performed by pushing out the staples towards the tissue all at once. Because the coil member does not buckle, the plurality of staples can be easily pushed out all at once by applying a large force.

In the above-described first and second aspects, at least two sets of the guide wire and the coil member may be provided with a space interposed therebetween in a direction orthogonal to the moving direction of the movable member.

By doing so, because the movable member is pressed simultaneously at a plurality of points by the coil members arranged in the form of a plurality of sets, the movable member can be moved more smoothly as a result of being prevented from tilting.

In the above-described second aspect, the movable members may be provided independently of one another so that the staples and the cutter are pushed individually.

In the above-described second aspect, the movable member may be provided so as to push the staples and the cutter simultaneously.

### {Advantageous Effects of Invention}

The present invention affords an advantage in that treatment, such as cutting off and stapling, of tissue can be performed easily and reliably without making the distal end portion large.

### {Brief Description of Drawings}

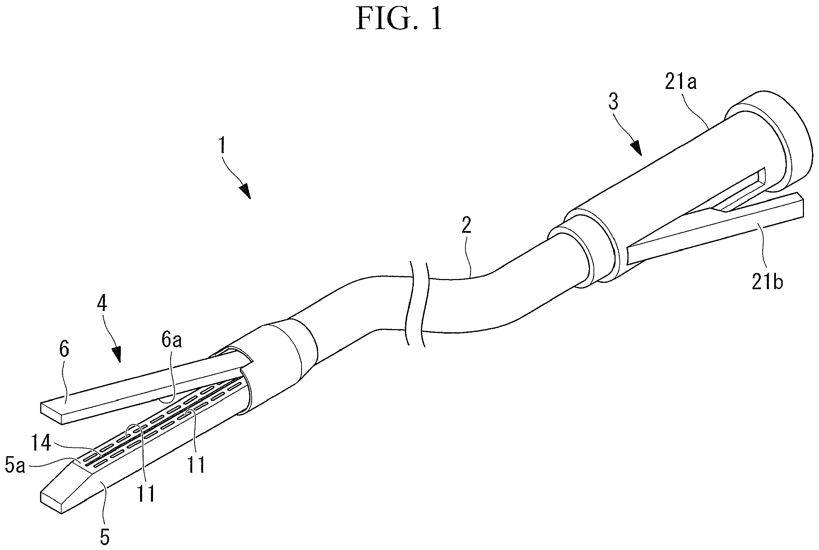

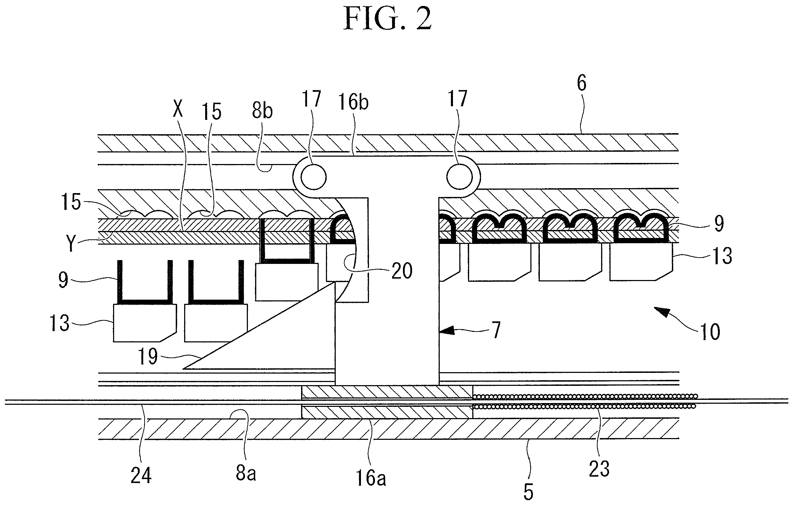

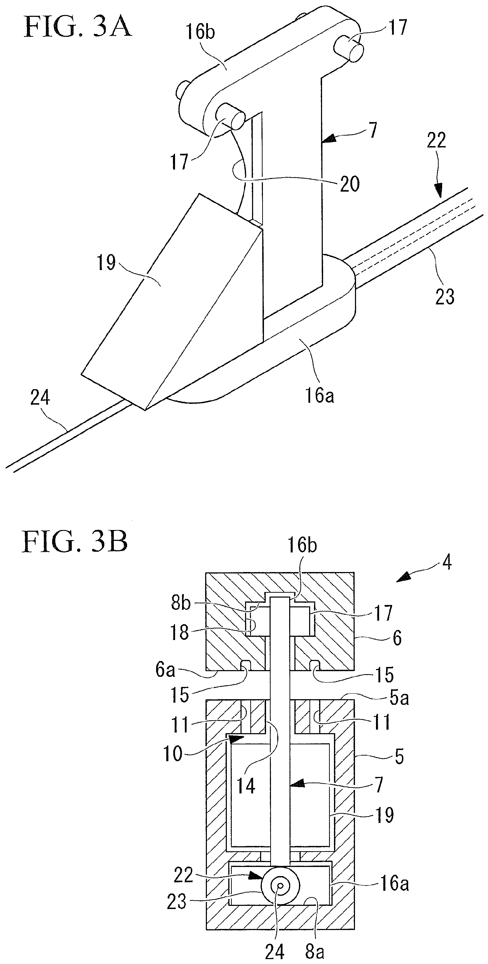

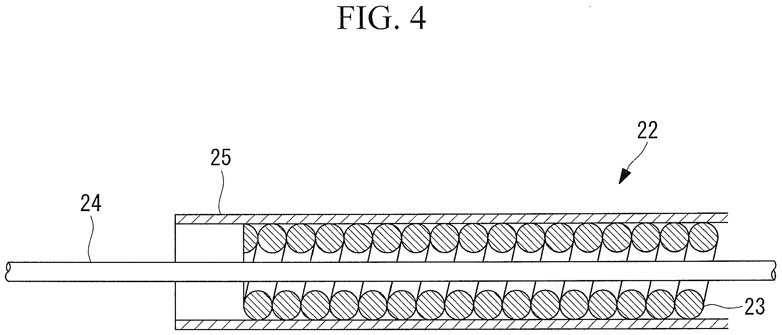

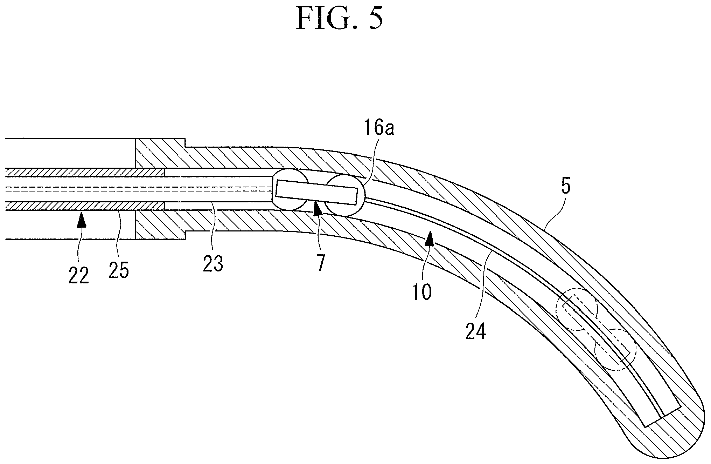

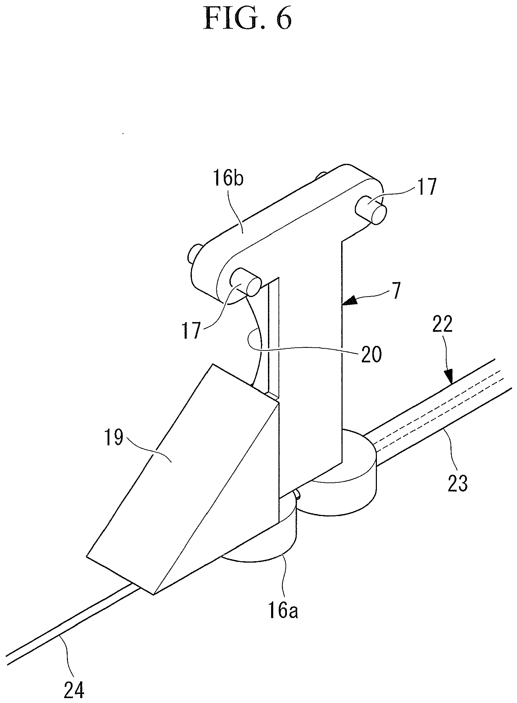

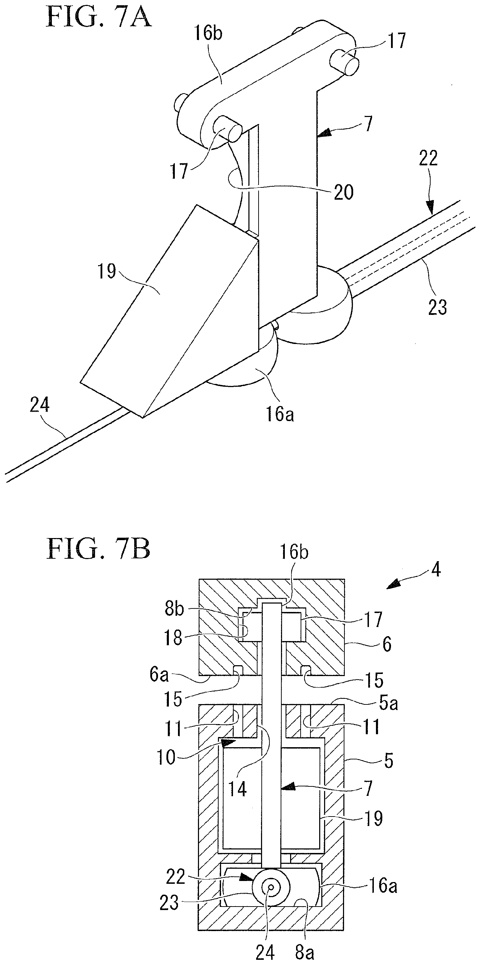

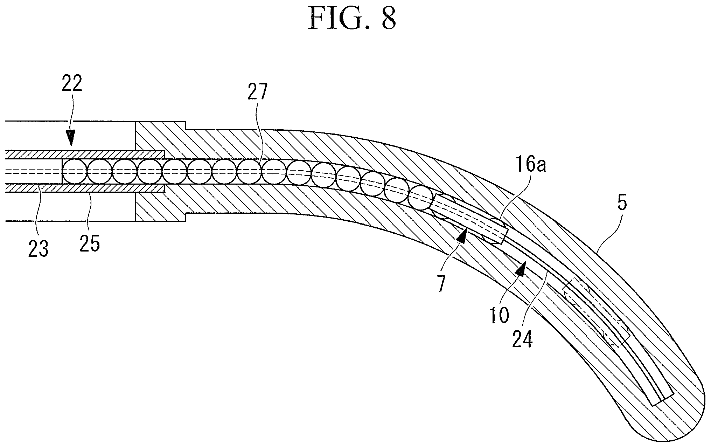

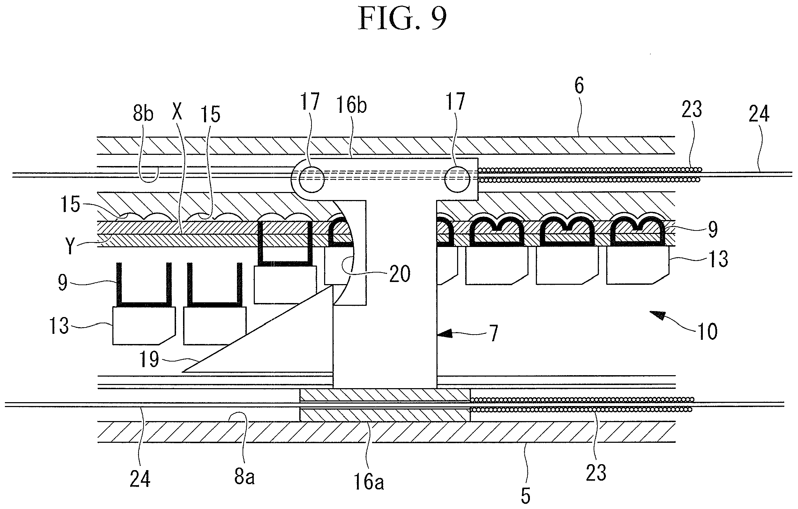

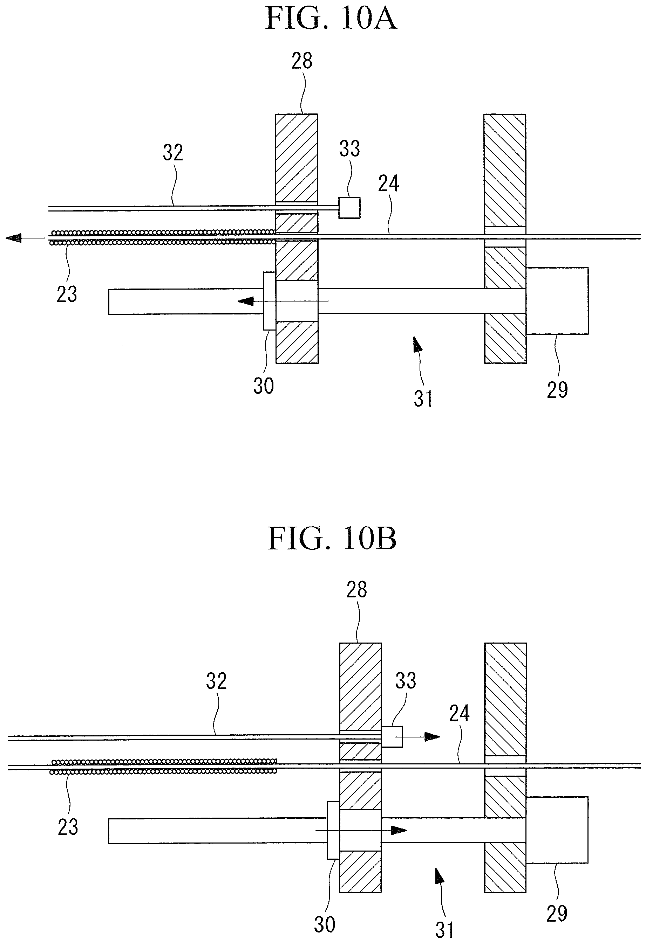

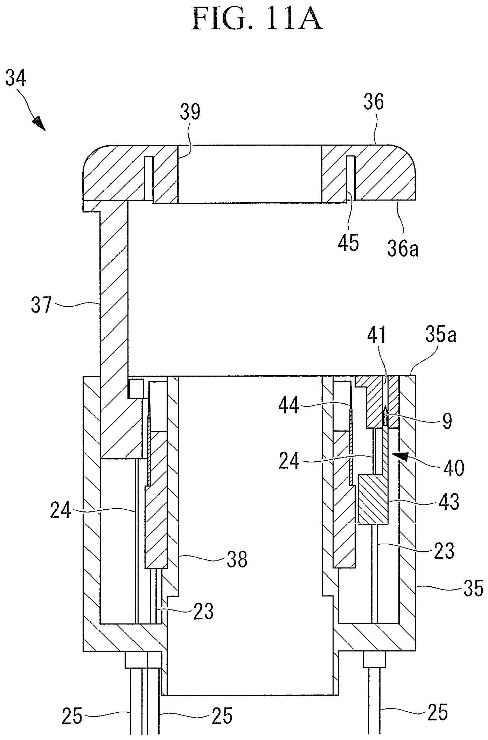

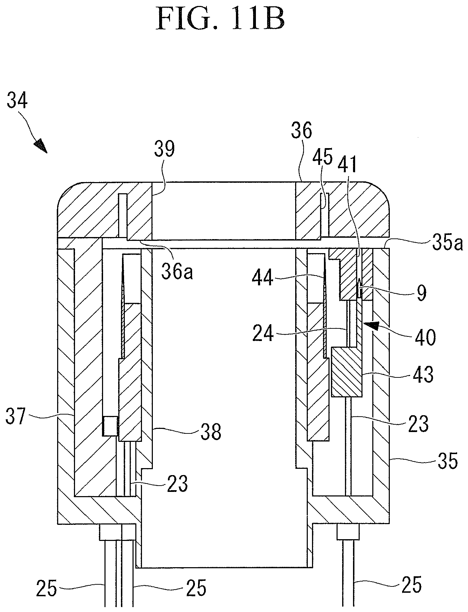

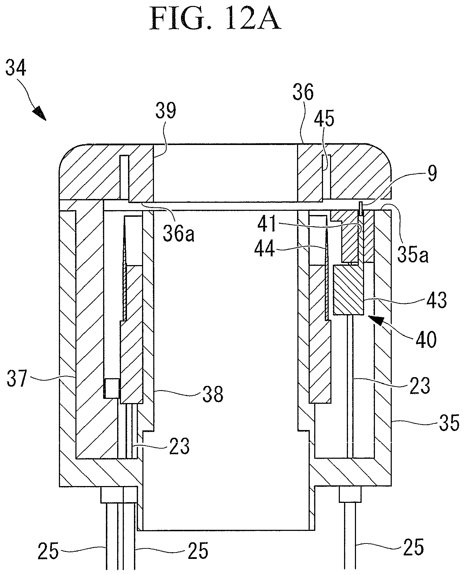

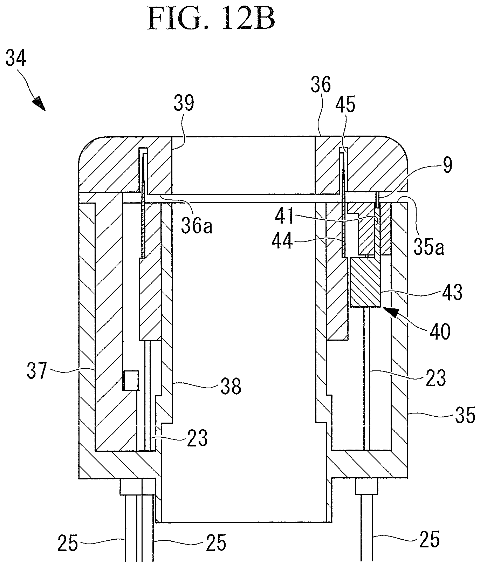

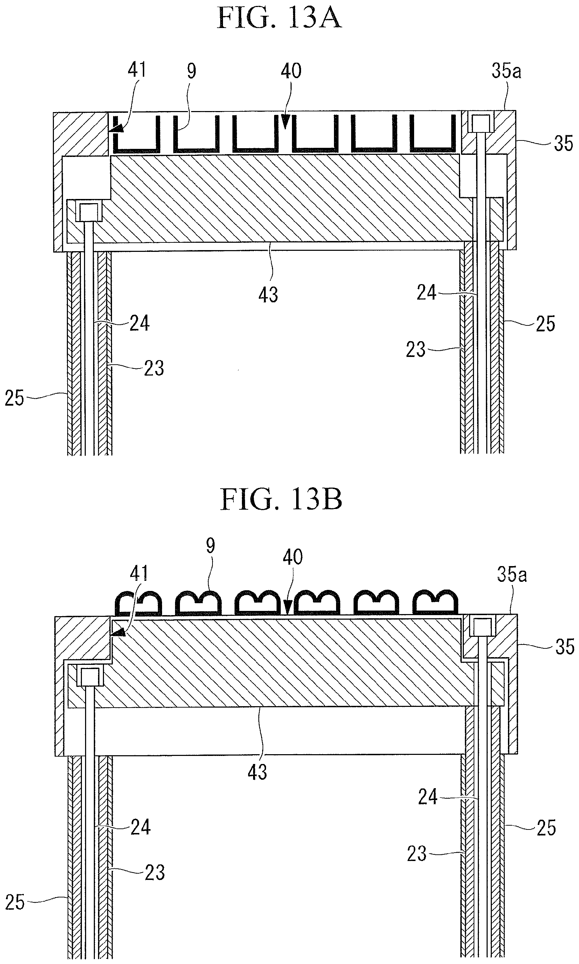

{Fig. 1} Fig. 1 is a perspective view showing a treatment device according to a first embodiment of the present invention.
{Fig. 2} Fig. 2 is a longitudinal sectional view showing a part of a stitching section of the treatment device in Fig. 1.
{Fig. 3A} Fig. 3A is a perspective view showing a movable member provided in the stitching section of the treatment device in Fig. 1.
{Fig. 3B} Fig. 3B is a transverse view of the stitching section provided with the movable member in Fig. 3A.
{Fig. 4} Fig. 4 is a longitudinal sectional view showing a coil member, a guide wire, and a hollow tube of the treatment device in Fig. 1.
{Fig. 5} Fig. 5 is a modification of the treatment device in Fig. 1, showing a longitudinal sectional view of the stitching section for illustrating the relationship between a guide groove and a slider section in a case where the stitching section is curved.
{Fig. 6} Fig. 6 is a perspective view showing the movable member having the slider section in Fig. 5.
{Fig. 7A} Fig. 7A is a perspective view showing the movable member having a slider section with a different shape from that in Fig. 6.
{Fig. 7B} Fig. 7B is a transverse view of the stitching section having the movable member in Fig. 7A.
{Fig. 8} Fig. 8 is a longitudinal sectional view of the stitching section having a pressing member composed of a plurality of spheres arranged between the slider section and the coil member in Fig. 5.
{Fig. 9} Fig. 9 is a longitudinal sectional view showing a modification of the stitching section in Fig. 2.
{Fig. 10A} Fig. 10A is a longitudinal sectional view showing an example of a mechanism in an operating section while stitching treatment is carried out by pressing the coil member.
{Fig. 10B} Fig. 10B is a longitudinal sectional view showing an example of the mechanism in the operating section while the movable member is pulled towards the basal end side.
{Fig. 11A} Fig. 11A is a longitudinal sectional view of a distal end portion showing a state where a second clamp member is open relative to a first clamp member in a treatment device according to a second embodiment of the present invention.
{Fig. 11B} Fig. 11B is a longitudinal sectional view of the distal end portion showing a state where the second clamp member is closed relative to the first clamp member in the treatment device according to the second embodiment of the present invention.
{Fig. 12A} Fig. 12A is a longitudinal sectional view of the distal end portion showing a state where a pusher is pushed from the state in Fig. 11B.
{Fig. 12B} Fig. 12B is a longitudinal sectional view of the distal end portion showing a state where a cutter is pushed out from the state in Fig. 11B.
{Fig. 13A} Fig. 13A is a schematic diagram of the distal end portion showing a state before staples are pushed out in the treatment device in Figs. 11A and 11B.
{Fig. 13B} Fig. 13B is a schematic diagram of the distal end portion showing a state where the staples are pushed out in the treatment device in Figs. 11A and 11B.

### {Description of Embodiments}

A treatment device 1 according to a first embodiment of the present invention will now be described with reference to the drawings.

As shown in Fig. 1, the treatment device 1 according to this embodiment includes an elongated insertion section 2 having flexibility; an operating section 3 connected to the basal end side of the insertion section 2; and a stitching section 4 connected to the distal end side of the insertion section 2.

As shown in Fig. 1, the stitching section 4 includes a first clamp member 5 and a second clamp member 6 that is provided so as to be openable and closable. The first clamp member 5 is fixed to the distal end of the insertion section 2. The second clamp member 6 is provided so as to be swivelable relative to the first clamp member 5 about an axial line orthogonal to the longitudinal axis of the insertion section 2. The stitching section 4 is closed by swiveling the second clamp member 6 in a direction towards the first clamp member 5, and the stitching section 4 is opened by swiveling the second clamp member 6 in a direction away from the first clamp member 5.

The first clamp member 5 and the second clamp member 6 are formed in the shape of a straight line and include guide grooves 8a and 8b for guiding a movable member 7, which will be described later, along the longitudinal directions thereof.

The first clamp member 5 also includes a cartridge section 10 for accommodating a plurality of staples 9. The cartridge section 10 includes a plurality of slots 11 formed as openings in a grip surface 5a, which faces the second clamp member 6 when the second clamp member 6 is closed relative to the first clamp member 5, and the slots 11 are arranged with spaces interposed therebetween in the longitudinal direction.

As shown in Fig. 2, each of the slots 11 accommodates the U-shaped staple 9 having a leading edge oriented towards the opening and a pusher 13 for pushing the staple 9 out of the slot 11. The pusher 13 is accommodated in the slot 11 so as to be movable in a direction towards the opening, and the staple 9 is disposed in contact with the pusher 13.

As shown in Fig. 1, the grip surface 5a of the first clamp member 5 includes a rectilinear slit 14 that extends in the longitudinal direction thereof and that allows the movable member 7, which will be described later, to pass therethrough such that the movable member 7 can move in the longitudinal direction thereof. In the example shown in Fig. 1, the slots 11 are arranged in two rows with the slit 14 interposed therebetween.

A grip surface 6a of the second clamp member 6 includes a plurality of recessed sections 15 for bending the distal ends of the staples 9.

As shown in Fig. 2, the stitching section 4 includes the movable member 7, which is movable towards the distal end of the stitching section 4 in a state where the second clamp member 6 is closed relative to the first clamp member 5.

As shown in Figs. 3A and 3B, the movable member 7 includes slider sections 16a and 16b that are inserted and disposed in the guide grooves 8a and 8b formed in the first clamp member 5 and the second clamp member 6, respectively.

The slider section 16a accommodated in the guide groove 8a of the first clamp member 5 has a transversal shape substantially equivalent to the transversal shape of the guide groove 8a, is formed in the shape of a rectangular column extending along the guide groove 8a, and has a shape resulting from chamfering both ends in the longitudinal direction. The slider section 16b accommodated in the guide groove 8b of the second clamp member 6 supports rods 17 extending in the width direction at two positions with a space interposed therebetween in the longitudinal direction and is slid along the guide groove 8b in a state where the rods 17 are accommodated in side grooves 18 formed in the side walls of the guide groove 8b. As a result of the rods 17 being accommodated in the side grooves 18, it is possible to prevent the second clamp member 6 from being opened relative to the first clamp member 5 during stapling.

In addition, the movable member 7 includes a wedge member 19 having a bevel on the distal end side and a cutter 20 having a leading edge oriented towards the distal end side. The bevel of the wedge member 19 is disposed at a position where a force for pushing each of the pushers 13 out of the slot 11 is produced by causing the pusher 13 to ride on the bevel when the movable member 7 is moved towards the distal end side. The leading edge of the cutter 20 is disposed at a position towards the basal end side from the bevel of the wedge member 19 so that tissues X and Y can be cut after the tissues X and Y have been stapled by the staple 9 pushed out of each of the slots.

The operating section 3 includes a handle 21a gripped by an operator and a lever 21b provided on that handle 21a so as to be swivelable. When the operator grips the handle 21a and lever 21b and applies a gripping force, the lever 21b swivels relative to the handle 21a, thereby making it possible to produce a pressing force for carrying out stitching motion with the stitching section 4.

The treatment device 1 according to this embodiment is characterized by a driving mechanism 22 for driving the movable member 7 with a pressing force applied via the operating section 3. As shown in Fig. 4, the driving mechanism 22 includes: a coil member 23 that is disposed between the operating section 3 and the stitching section 4 so as to pass through the insertion section 2 to transmit, in the longitudinal direction, the pressing force produced in the operating section 3; a guide wire 24 for guiding the coil member 23; and a hollow tube 25.

The coil member 23 is a coil tube formed of a tightly wound coil spring and not only is easily bent but also has compressive resistance capable of transmitting a large pressing force applied in the longitudinal direction.

The basal end of the coil member 23 is in contact with a pressing rod (not shown in the figure). The pressing rod is driven by a transformation mechanism (not shown in the figure) for transforming the swiveling movement of the lever 21b into a longitudinal advancement/withdrawal motion and is pushed towards the distal end side when the lever 21b is gripped, thereby applying a pressing force to the coil member 23.

As shown in Fig. 2, the distal end of the coil member 23 is accommodated in the guide groove 8a of the first clamp member 5 and is in contact with the basal end of the slider section 16a of the movable member 7. By doing so, the pressing force applied in the operating section 3 is transmitted via the coil member 23 to press the slider section 16a, thus making it possible to push the movable member 7 towards the distal end side.

The guide wire 24 is a metal wire having flexibility and is disposed so as to pass through the coil member 23 in the longitudinal direction. The distal end of the guide wire 24 is fixed in the vicinity of the distal end of the first clamp member 5 through a through-hole provided in the slider section 16a, and the basal end is fixed to the handle 21a of the operating section 3. While supporting the coil member 23 so as to be movable in the longitudinal direction, the guide wire 24 supports the coil member 23 from the radially inner side, thereby protecting the coil member 23 so as not to buckle despite an excessive pressing force acting on the coil member 23.

The hollow tube 25 is a resin tube having flexibility. The hollow tube 25 accommodates the coil member 23 disposed in the insertion section 2 such that the coil member 23 can move in the longitudinal direction to prevent the coil member 23 from being deformed radially outward when a pressing force is applied to the coil member 23.

The operation of the treatment device 1 according to this embodiment with this structure will be described below.

In order to stitch the tissues X and Y using the treatment device 1 according to this embodiment, the insertion section 2 is inserted into a hole provided in the abdominal wall or into the body cavity from the stitching section 4 side, and then the stitching section 4 is placed at a site to be stitched. In this process, the insertion section 2 having flexibility is bent in accordance with the shape and so forth of the organ, and the guide wire 24, the coil member 23, and the hollow tube 25 passing through the inside are also bent in the same manner.

In this state, the two tissues X and Y to be stitched are arranged so as to be overlaid between the first clamp member 5 and the second clamp member 6, the handle 21a and the lever 21b of the operating section 3 are gripped, and a gripping force is applied to produce a pressing force.

The produced pressing force is transmitted to the stitching section 4 through the coil member 23, thus moving the movable member 7 provided in the stitching section 4 towards the distal end.

When the movable member 7 is moved towards the distal end, the rods 17 provided in the slider section 16b of the movable member 7 mesh with the side grooves 18 in the guide groove 8b of the second clamp member 6, and as a result of the second clamp member 6 being drawn, the second clamp member 6 is swiveled relative to the first clamp member 5 in a direction to narrow the space therebetween. By doing so, the stitching section 4 is closed, and the two tissues X and Y to be stitched are sandwiched between the first clamp member 5 and the second clamp member 6 in the thickness direction.

By further increasing, from this state, the gripping force to be applied to the lever 21b, the movable member 7 is further pushed towards the distal end side. By doing so, the bevel of the wedge member 19 fixed to the movable member 7 sequentially pushes the pushers 13 in a direction orthogonal to the moving direction of the movable member 7, causing the staples 9 in the slots 11 to be pushed out of the openings of the slots 11 formed in the grip surface 5a. The pushed out staples 9 are pushed into the tissues X and Y clamped by the grip surfaces 5a and 6a, and the distal ends of the staples 9 are deformed by means of the recessed sections 15 of the second clamp member 6 that is disposed so as to face the openings of the slots 11, whereby the two tissues X and Y are stitched in two rows so as not to detach from each other.

Furthermore, immediately after the tissues X and Y are stitched by the staples 9, the cutter 20 disposed on the distal end side of the movable member 7 cuts the tissues X and Y between the sites that have been stitched by the two rows of staples 9. By doing so, the two tissues X and Y are cut off near the cut surface, thus completing stitching treatment in a state in which they are stapled along the cut surface.

In this manner, according to the treatment device 1 of this embodiment, because the pressing force produced in the operating section 3 is transmitted to the stitching section 4 by the coil member 23 having flexibility, the pressing force can be reliably transmitted to the stitching section 4 even in a state where the insertion section 2, which is bendable and flexible, is curved. In this case, unlike a conventional treatment device in which a pressing force is produced by folding back a wire by means of a pulley, it is not necessary to provide a pulley at the distal end of the stitching section 4, thereby making it possible to form the stitching section 4 with a thin configuration. Consequently, this provides an advantage in that the ease of insertion into the body is enhanced and stress to the patient is reduced.

In this embodiment, the slots 11 are arranged in two rows with the slit 14 interposed therebetween. However, the invention is not limited to this, and the slots 11 may be arranged in one row or three or more rows.

This embodiment has been described by way of an example where the first clamp member 5 and the second clamp member 6 of the stitching section 4 have a straight line shape. However, the first clamp member 5 and the second clamp member 6 may have a shape curved in one direction along the longitudinal direction as shown in Fig. 5.

In this case, for the slider section 16a accommodated in the guide groove 8a provided in the first clamp member 5, it is preferable to employ a slider section 16a that has a cylindrical surface, as shown in Fig. 6, or a spherical surface, as shown in Figs. 7A and 7B, so that the slider section 16a comes into contact with both side walls of the guide groove 8a at two points with a space interposed therebetween in the longitudinal direction.

By doing so, the slider section 16a can be slid continuously along a guide groove from a rectilinear guide groove to a curved guide groove.

This embodiment has been described by way of an example where the slider section 16a is pressed directly by the coil member 23. Instead of this, a pressing member 27 in the form of a string of beads, which is composed of a plurality of spheres arranged in a row and through which the guide wire 24 passes, may be disposed between the coil member 23 and the slider section 16a, as shown in Fig. 8.

This embodiment has been described by way of an example where the slider section 16a disposed in the guide groove 8a of the first clamp member 5 is pressed by the coil member 23, a guide wire 24 may also be made to pass through the slider section 16b disposed in the guide groove 8b of the second clamp member 6 so that the guide wire 24 may be pressed by a coil member 23, as shown in Fig. 9.

By doing so, it is possible to prevent the movable member 7 from swinging/tilting (falling), which is caused by a reaction force that is applied to the cutter 20 when the tissues X and Y are cut.

In this embodiment, the mechanism for applying a pressing force via the operation of the operating section 3 may be realized by a mechanism 31 in which a plate 28 in contact with the basal end side of the coil member 23 is moved with a motor 29 and a ball screw 30 in the longitudinal direction (direction indicated by the arrow) of the coil member 23, as shown in Fig. 10A. In this case, a separate wire 32 for pulling the movable member 7 towards the basal end side is preferably provided, as shown in Fig. 10B, in order to return the movable member 7 to the basal end side and open the second clamp member 6 relative to the first clamp member 5 after stitching of the tissues X and Y has completed. The distal end of the wire 32 is fixed to the movable member 7, and the basal end of the wire 32 is provided with a large diameter section 33 brought into engagement with the plate 28.

More specifically, when the movable member 7 is to be moved towards the distal end side, the ball screw 30 is rotated in one direction so that the coil member 23 is pressed by the plate 28, as shown in Fig. 10A. On the other hand, when the movable member 7 is to be pulled towards the basal end side, the ball screw 30 is rotated in the reverse direction to move the plate 28 in a direction away from the coil member 23, as shown in Fig. 10B. Because the large diameter section 33 provided at the basal end of the wire 32 is brought into engagement with the plate 28 so that a traction force acts on the wire 32, the movable member 7 can be pulled towards the basal end side.

This affords an advantage in that the coil member 23 can be relieved from a traction force, thereby maintaining the coil member 23 in a sound state.

This embodiment has been described by way of an example where the movable member 7 is pressed with the single coil member 23. Instead of this, the movable member 7 may be pressed with two or more coil members 23 if a sufficient space is available. By doing so, the pressing force due to each of the coil members 23 can be distributed.

This embodiment has been described by way of an example of the treatment device 1 employing a method for pressing the wedge member 19 by pressing the movable member 7 with the coil member 23, thereby pushing out the staples 9 with the pushers 13. Instead of this, the invention may be applied to staples 9 employing a circular method. In this case, it is advisable that the pushers 13 be pressed directly by the coil member 23. In this case, it is effective to press the pushers 13 by distributing a pressing force among a plurality of the coil members 23.

This embodiment has been described by way of an example of the stitching section 4 configured to be opened/closed by swiveling the second clamp member 6 relative to the first clamp member 5. However, the invention may be applied to a stitching section 4 configured to be opened/closed via translational movement of the second clamp member 6 and to a stitching section 4 configured to be closed in a half-open state.

The above-described first embodiment and modifications thereof have been described by way of a treatment device employing a linear method, and the staples 9 and the cutter 20 are based on the method for driving the common movable member 7 using the guide wire 24 and the coil member 23.

Next, a treatment device according to a second embodiment of the present invention will be described with reference to the drawings.

In the description of this embodiment, the structures common to those used in the treatment device 1 according to the above-described first embodiment are denoted by the same reference signs, and a description thereof will be omitted.

The treatment device according to this embodiment differs from the treatment device 1 according to the first embodiment in that the treatment device according to this embodiment is a treatment device employing a so-called circular method in which a first clamp member 35 is disposed on an arc along a flat surface orthogonal to the longitudinal direction of the insertion section 2, whereas the treatment device 1 according to the first embodiment employs a so-called linear method in which the first clamp member 5 is disposed along the longitudinal direction of the insertion section 2.

More specifically, the treatment device employing the circular method includes a stitching section 34 having the cylindrical first clamp member 35 and a ring-shaped second clamp member 36, as shown in Figs. 11A and 11B. The second clamp member 36 is disposed on the distal end side of the first clamp member 35 and is provided so as to be movable in the axial direction with a guide member 37. The space between the clamp members 35 and 36 is made narrow by pulling the second clamp member 36 towards the basal end side relative to the first clamp member 35, so that the tissues X and Y can be sandwiched between the clamp members 35 and 36. The reason why the first clamp member 35 has a cylindrical shape and the second clamp member 36 has a ring shape in this embodiment is to allow, for example, an endoscope to pass through through-holes 38 and 39 in the centers thereof.

The first clamp member 35 includes a cartridge section 40 for accommodating a plurality of staples 9. The cartridge section 40 is provided with a plurality of slots 41 formed as openings in a grip surface 35a, which faces the second clamp member 36 when the second clamp member 36 is closed relative to the first clamp member 35, such that the slots 41 are arranged with spaces interposed therebetween in the circumferential direction.

As shown in Fig. 13A, each of the slots 41 accommodates a U-shaped staple 9 having a leading edge oriented towards the opening and a pusher 43 for pushing the staple 9 out of the slot 41. The pusher 43 is accommodated in the slots 41 so as to be movable in a direction towards the openings, and the staples 9 are disposed in contact with the pusher 43.

As shown in Figs. 11A to 12B, a cylindrical cutter 44 is configured to be capable of appearing above and disappearing below the grip surface 35a of the first clamp member 35. A grip surface 36a of the second clamp member 36 is provided with a cylindrical slit 45 for accommodating the cutter 44 that projects from the first clamp member 35.

The grip surface 36a of the second clamp member 36 is provided with a plurality of recessed sections (not shown in the figure) for bending the distal ends of the staples 9.

The driving mechanism 22 for driving the pusher (movable member) 43 and the cutter (movable member) 44 with a pressing force applied via the operating section 3 includes: a coil member 23 for transmitting, in the longitudinal direction, the pressing force produced in the operating section 3; a guide wire 24 for guiding the coil member 23; and a hollow tube 25.

As shown in Fig. 11A, the distal end of the coil member 23 is in contact with the basal ends of the pusher 43 and the cutter 44. By doing so, the pressing force applied in the operating section 3 can be transmitted by means of the coil member 23, so that the pusher 43 and the cutter 44 can be pushed towards the distal end.

The distal end of the guide wire 24 that is disposed so as to pass through the coil member 23 in the longitudinal direction is fixed in the vicinity of the distal end of the first clamp member 35 through the pusher 43 and the cutter 44.

This embodiment includes three sets of: the coil member 23, the distal end of which is pressed against the basal end surface of the first clamp member 35; the guide wire 24, which passes through the coil member 23 in the longitudinal direction; and the hollow tube 25, which covers the coil member 23. The distal ends of the guide wires 24 are fixed to the guide member 37, the cutter 44, and the pusher 43, respectively, of the first clamp member 35.

The operation of the treatment device according to this embodiment with this structure will be described below.

As shown in Fig. 11A, in order to stitch the tissues X and Y, particularly a tubular tissue, using the treatment device according to this embodiment, the second clamp member 36 is moved forward relative to the first clamp member 35, and the tissues X and Y are sandwiched between the clamp members 35 and 36.

In this state, by applying a traction force to the guide wire 24 the distal end of which is fixed to the guide member 37, the second clamp member 36 is drawn towards the basal end side relative to the first clamp member 35, so that the tissues X and Y can be sandwiched between the grip surfaces 35a and 36a of the clamp members 35 and 36. At this time, while the traction force is transmitted to the guide member 37 using the guide wire 24, the second clamp member 36 can be reliably drawn towards the first clamp member 35, as shown in Fig. 11B, by holding the basal end side of the first clamp member 35 with the coil member 23 through which the guide wire 24 passes.

Next, by pushing forward the coil member 23 the distal end of which abuts against the pusher 43, the plurality of staples 9 in the slots 41 are pushed out of the slots 41 all at once by pushing the pusher 43. Because the distal ends of the pushed out staples 9 are bent in the recessed sections in the second clamp member 36, the tissues X and Y gripped between the clamp members 35 and 36 can be stitched in the circumferential direction.

In this case, according to this embodiment, a pressing force can be applied by means of the coil member 23 supported by the guide wire 24 from radially inside without buckling the coil member 23. Consequently, a large force that can push out the plurality of staples 9 all at once can be applied to the coil member 23, thereby making it possible to easily stitch the tissues X and Y.

Furthermore, by pushing forward the coil member 23 the distal end of which abuts against the cutter 44, the cutter 44 is pushed out to cut into a circular shape the tissues X and Y gripped between the clamp members 35 and 36. Also in this case, a large pressing force can be applied by means of the coil member 23 supported by the guide wire 24 from radially inside without buckling the coil member 23. Consequently, a large force for cutting both tissues X and Y at once along the entire circumference thereof can be applied to the coil member 23, thus making it possible to easily cut the tissues X and Y.

In this embodiment, the distal ends of the guide wires 24 are fixed to the pusher 43 and to the cutter 44, as shown in Figs. 13A and 13B, and the coil members 23 through which the guide wires 24 are made to pass in the longitudinal direction are made to abut against the basal end side of the first clamp member 35. Therefore, while holding the first clamp member 35 with the coil members 23 so that the first clamp member 35 does not move towards the basal end side, the pusher 43 and the cutter 44 can be pulled back towards the basal end side with the guide wires 24 by pulling the guide wires 24.

The above-described first and second embodiments have been described by way of examples where the treatment devices are provided with the staples 9 and the cutters 20 and 44. However, the invention is not limited to these examples but can be applied to a treatment device provided with either staples or a cutter.

In the above-described first and second embodiments, a puncturing needle for puncturing the tissues X and Y sandwiched between the pair of clamp members 5 and 6 may be provided. A puncturing needle may be provided instead of the cutters 20 and 44.

In this case, the coil member 23 is made to abut against the puncturing needle, and as a result of the coil member 23 being pushed forward, the tissues X and Y can be punctured with the puncturing needle.

### {Reference Signs List}

- 1: Treatment device
- 2: Insertion section
- 3: Operating section
- 4, 34: Stitching section
- 5, 35: First clamp member
- 6, 36: Second clamp member
- 7: Movable member
- 8a, 8b: Guide groove
- 9: Staple
- 16a, 16b: Slider section
- 22: Driving mechanism
- 23: Coil member
- 24: Guide wire
- 25: Hollow tube
- 43: Pusher (movable member)
- 44: Cutter (movable member)
- X, Y: Tissue

## Claims

1. A treatment device comprising:
an operating section operated by an operator;
an elongated insertion section that is connected to the operating section and that has flexibility;
a stitching section that is provided at a distal end of the insertion section and that included a first clamp member that accommodates a plurality of arranged staples and a second clamp member that is provided so as to be openable and closable relative to the first clamp member;
a movable member that is provided in the first clamp member of the stitching section and that is moved so as to push the staples relative to tissue sandwiched between the first clamp member and the second clamp member; and
a driving mechanism for moving the movable member towards a distal end side,
wherein the driving mechanism includes: a coil member that is provided so as to pass through the insertion section in a longitudinal direction, the distal end of which is brought into contact with the movable member, and that transmits a pressing force applied in the operating section;
and
a guide wire that passes through the coil member in the longitudinal direction, one end of which is fixed to the first clamp member and the other end of which is fixed to the operating section, and that guides the movement of the coil member.

2. A treatment device comprising:
an operating section operated by an operator;
an elongated insertion section that is connected to the operating section and that has flexibility;
a stitching section including a first clamp member that is provided at a distal end of the insertion section and that accommodates a plurality of arranged staples and a second clamp member that is provided so as to be openable and closable relative to the first clamp member;
a cutter for cutting tissue sandwiched between the first clamp member and the second clamp member of the stitching section;
a movable member that is provided in the first clamp member of the stitching section and that is moved so as to push either or both of the staples and the cutter relative to the tissue sandwiched between the first clamp member and the second clamp member;
a driving mechanism for moving the movable member towards a distal end side,
wherein the driving mechanism includes: a coil member that is provided so as to pass through the insertion section in a longitudinal direction, the distal end of which is brought into contact with the movable member, and that transmits a pressing force applied in the operating section;
and
a guide wire that passes through the coil member in the longitudinal direction, one end of which is fixed to the first clamp member and the other end of which is fixed to the operating section, and that guides the movement of the coil member.

3. The treatment device according to claim 1 or 2, comprising: a hollow tube that has flexibility and that allows the coil member to pass therethrough so as to be movable in the longitudinal direction in the insertion section.

4. The treatment device according to claim 1 or 2, wherein the first clamp member accommodates the plurality of staples such that the staples are arranged in the longitudinal direction of the insertion section, and
the movable member is moved towards the distal end side along the first clamp member so as to push out the staples sequentially.

5. The treatment device according to 4, wherein the first clamp member includes a guide groove for guiding the movement of the movable member.

6. The treatment device according to 5, wherein the movable member includes a slider section that slides in the guide groove, and
the slider section is shaped so as to be in contact with each side wall of the guide groove at two positions with a space interposed therebetween in the slide direction of the slider section.

7. The treatment device according to claim 1 or 2, wherein the first clamp member accommodates the plurality of staples such that the staples are arranged on an arc along a direction orthogonal to the longitudinal direction of the insertion section, and
the movable member is moved so as to push out the staples all at once.

8. The treatment device according to claim 1 or 2, wherein at least two sets of the guide wire and the coil member are provided with a space interposed therebetween in a direction orthogonal to the moving direction of the movable member.

9. The treatment device according to 2, wherein the movable members are provided independently of one another so that the staples and the cutter are pushed individually.

10. The treatment device according to 2, wherein the movable member is provided so as to push the staples and the cutter simultaneously.
